(19) Europäisches Patentamt
European Patent Office
Office européen des brevets

(11) Veröffentlichungsnummer : **0 135 182**
**B1**

(12) # EUROPÄISCHE PATENTSCHRIFT

(45) Veröffentlichungstag der Patentschrift : **27.07.88**

(51) Int. Cl.[4] : **C 07 K 5/06**, C 07 K 1/08, A 61 K 37/02

(21) Anmeldenummer : **84110678.4**

(22) Anmeldetag : **07.09.84**

(54) **Verfahren zur Herstellung von N-alkylierten Dipeptiden und deren Estern.**

(30) Priorität : **16.09.83 DE 3333454**

(43) Veröffentlichungstag der Anmeldung : **27.03.85 Patentblatt 85/13**

(45) Bekanntmachung des Hinweises auf die Patenterteilung : **27.07.88 Patentblatt 88/30**

(84) Benannte Vertragsstaaten : **AT BE CH DE FR GB IT LI LU NL SE**

(56) Entgegenhaltungen :
**EP-A- 0 049 605**
**EP-A- 0 079 022**
**EP-A- 0 088 342**
**EP-A- 0 089 637**
**DE-A- 2 901 843**
**DE-A- 3 315 464**
**Arzneimittelforschung 34(II), Nr. 10b(1984), 1399-1401**

(73) Patentinhaber : **HOECHST AKTIENGESELLSCHAFT**
**Postfach 80 03 20**
**D-6230 Frankfurt am Main 80 (DE)**

(72) Erfinder : **Teetz, Volker, Dr.**
**An der Tann 20**
**D-6238 Hofheim am Taunus (DE)**
Erfinder : **Wissmann, Hans, Dr.**
**Falkenstrasse 12**
**D-6232 Bad Soden am Taunus (DE)**
Erfinder : **Urbach, Hansjörg, Dr.**
**Le Lavandoustrasse 41**
**D-6242 Kronberg/Taunus (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 135 182 B1

**Beschreibung**

Aus der EP-A-56 618 ist ein Verfahren zur Herstellung von Carbonsäureamidgruppen enthaltenden Verbindungen durch Umsetzung von COOH-Gruppen enthaltenden Verbindungen mit Verbindungen, die eine freie $NH_2$-Gruppe enthalten, in Gegenwart von Dialkylphosphinsäureanhydriden bekannt.

Gemäß den Angaben in dieser EP-A, müssen dabei nicht an der Reaktion beteiligte Aminogruppen in den Ausgangsmaterialien durch Schutzgruppen blockiert sein.

Es wurde nun gefunden, daß sich bestimmte N-alkylierte Dipeptide und deren Ester durch Kupplung einer Carbonsäure mit einem sekundären Amin in Gegenwart von Dialkylphosphinsäureanhydriden herstellen lassen, ohne daß es dabei erforderlich ist, nicht an der Reaktion beteiligte NH-Gruppen durch Schutzgruppen zu blockieren.

Die Erfindung betrifft daher ein Verfahren zur Herstellung von Verbindungen der Formel I

$$R^3OOC-\underset{R^4}{\overset{*}{C}H}-\underset{R^5}{N}-\underset{O}{\underset{\|}{C}}-\underset{R^1}{\overset{*}{C}H}-NH-\underset{COOR^2}{\overset{*}{C}H}-(CH_2)_n-R \qquad (I)$$

in welcher

$n = 1$ oder 2 ist,

R Wasserstoff, Alkyl mit 1-8 C-Atomen, Alkenyl mit 2-6 C-Atomen, Cycloalkyl mit 3-9 C-Atomen, Aryl mit 6-12 C-Atomen, das durch $(C_1-C_4)$-Alkyl, $(C_1-C_3)$-Alkoxy, Hydroxy, Halogen, Nitro, Amino, Aminomethyl, $(C_1-C_4)$-Alkylamino, Di-$(C_1-C_4)$-alkylamino, $(C_1-C_4)$-Acylamino, vorzugsweise $(C_1-C_4)$-Alkanoylamino, Methylendioxy, Carboxy, Cyano und/oder Sulfamoyl mono-, di- oder trisubstituiert sein kann, Alkoxy mit 1-4 C-Atomen, Aryloxy mit 6-12 C-Atomen, das wie oben bei Aryl beschrieben substituiert sein kann, mono- bzw. bicyclisches Heteroaryloxy mit 5-7 bzw. 8-10 Ringatomen, wovon 1 bis 2 Ringatome Schwefel- oder Sauerstoffatome und/oder 1 bis 4 Ringatome Stickstoff darstellen, das wie oben bei Aryl beschrieben substituiert sein kann, Amino-$(C_1-C_4)$-alkyl, $(C_1-C_4)$-Alkanoylamino-$(C_1-C_4)$-alkyl, $(C_7-C_{13})$-Aroylamino-$(C_1-C_4)$-alkyl, $(C_1-C_4)$-Alkoxy-carbonylamino-$(C_1-C_4)$-alkyl, $(C_6-C_{12})$-Aryl-$(C_1-C_4)$-alkoxycarbonylamino-$(C_1-C_4)$-alkyl, $(C_6-C_{12})$-Aryl-$(C_1-C_4)$-alkylamino-$(C_1-C_4)$-alkyl, $(C_1-C_4)$-Alkylamino-$(C_1-C_4)$-alkyl, Di-$(C_1-C_4)$-alkylamino-$(C_1-C_4)$-alkyl, Guanidino-$(C_1-C_4)$-alkyl, Imidazolyl, Indolyl, $(C_1-C_4)$-Alkylthio, $(C_1-C_4)$-Alkylthio-$(C_1-C_4)$-alkyl, $(C_6-C_{12})$-Arylthio-$(C_1-C_4)$-alkyl, das im Arylteil wie oben bei Aryl beschrieben, substituiert sein kann, $(C_6-C_{12})$-Aryl-$(C_1-C_4)$-alkylthio, das im Arylteil wie oben bei Aryl beschrieben substituiert sein kann, Carboxy-$(C_1-C_4)$-alkyl, Carboxy, Carbamoyl, Carbamoyl-$(C_1-C_4)$-alkyl, $(C_1-C_4)$-Alkoxy-carbonyl-$(C_1-C_4)$-alkyl, $(C_6-C_{12})$-Aryloxy-$(C_1-C_4)$-alkyl, das im Arylteil wie oben bei Aryl beschrieben substituiert sein kann oder $(C_6-C_{12})$-Aryl-$(C_1-C_4)$-alkoxy, das im Arylteil wie oben bei Aryl beschrieben substituiert sein kann,

$R^1$ Wasserstoff, Alkyl mit 1-6 C-Atomen, Alkenyl mit 2-6 C-Atomen, Alkinyl mit 2-6 C-Atomen, Cycloalkyl mit 3-9 C-Atomen, Cycloalkenyl mit 5-9 C-Atomen, $(C_3-C_9)$-Cycloalkyl-$(C_1-C_4)$-alkyl, $(C_5-C_9)$-Cycloalkenyl$(C_1-C_4)$-alkyl, gegebenenfalls teilhydriertes Aryl mit 6-12 C-Atomen, das wie oben bei R beschrieben substituiert sein kann, $(C_6-C_{12})$-Aryl-$(C_1-C_4)$-alkyl oder $(C_7-C_{13})$-Aroyl-($C_1$ oder $C_2$)-alkyl die beide wie das vorstehende Aryl substituiert sein können, mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann oder die gegebenenfalls geschützte Seitenkette einer natürlich vorkommenden α-Aminosäure $R^1$—$CH(NH_2)$—$COOH$ bedeuten,

$R^2$ und $R^3$ gleich oder verschieden sind und Wasserstoff, Alkyl mit 1-6 C-Atomen, Alkenyl mit 2-6 C-Atomen, Di-$(C_1-C_4)$-alkylamino-$(C_1-C_4)$-alkyl, $(C_1-C_5)$-Alkanoyloxy-$(C_1-C_4)$-alkyl, $(C_1-C_6)$-Alkoxy-carbonyloxy-$(C_1-C_4)$-alkyl, $(C_7-C_{13})$-Aroyloxy-$(C_1-C_4)$-alkyl, $(C_6-C_{12})$-Aryloxycarbonyloxy-$(C_1-C_4)$-alkyl, Aryl mit 6-12 C-Atomen, $(C_6-C_{12})$-Aryl-$(C_1-C_4)$-alkyl, $(C_3-C_9)$-Cycloalkyl oder $(C_3-C_9)$-Cycloalkyl-$(C_1-C_4)$-alkyl bedeuten und

$R^4$ und $R^5$ zusammen mit den sie tragenden Atomen ein heterocyclisches mono-, bi- oder tricyclisches Ringsystem mit 3 bis 15 C-Atomen bilden, das dadurch gekennzeichnet ist, daß man Verbindungen der Formel II

$$HO-\underset{O}{\underset{\|}{C}}-\underset{R^1}{CH}-NH-\underset{COOR^2}{CH}-(CH_2)_n-R \qquad (II)$$

in welcher n, R, $R^1$ und $R^2$ die oben definierten Bedeutungen haben, in Gegenwart von Phosphinsäureanhydriden der Formel III

$$O = \underset{R^7}{\overset{R^6}{\underset{|}{\overset{|}{P}}}} - O - \underset{R^9}{\overset{R^8}{\underset{|}{\overset{|}{P}}}} = O \qquad \text{(III)}$$

in welcher $R^6$, $R^7$, $R^8$ und $R^9$ gleich oder verschieden sind und für Alkyl und/oder Aralkyl stehen, mit Verbindungen der Formel IV

$$R^3OOC - \underset{R^4}{\underset{|}{CH}} - \underset{R^5}{\underset{|}{NH}} \qquad \text{(IV)}$$

in welcher $R^3$, $R^4$ und $R^5$ die oben definierten Bedeutungen haben, umsetzt, gegebenenfalls zum Schutz anderer funktioneller Gruppen eingeführte Reste abspaltet und gegebenenfalls freie Carboxygruppen in an sich bekannter Weise verestert.

Als solche Ringsysteme kommen insbesondere jene aus der folgenden Gruppe in Betracht :

Pyrrolidin (A) ; Piperidin (B) ; Tetrahydroisochinolin (C) ; Decahydroisochinolin (D) ; Octahydroindol (E) ; Octahydrocyclopenta [b] pyrrol (F) ; 2-Aza-bicyclo [2.2.2.] octan (G) ; 2-Azabicyclo [2.2.1] heptan (H) ; 2-Azaspiro [4.5] decan (I) ; 2-Azaspiro [4.4] nonan (J) ; Spiro [(bicyclo [2.2.1] heptan)-2,3-pyrrolidin] (K) ; Spiro [(bicyclo [2.2.2] octan)-2,3-pyrrolidin] (L) ; 2-Azatricyclo [4.3.0.1$^{6,9}$] decan (M) ; Decahydrocyclohepta [b] pyrrol (N) ; Octahydroisoindol (O) ; Octahydrocyclopenta [c] pyrrol (P) ; 2,3,3a,4,5,7a-Hexahydroindol (Q) ; Tetrahydrothiazol (R) ; 2-Azabicyclo [3.1.0] hexan (S) ; die alle gegebenenfalls substituiert sein können. Bevorzugt sind jedoch die unsubstituierten Systeme.

Die in Betracht kommenden cyclischen Aminosäureester weisen die folgenden Strukturformeln auf.

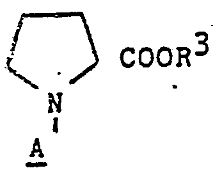
A

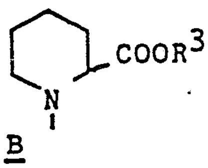
B

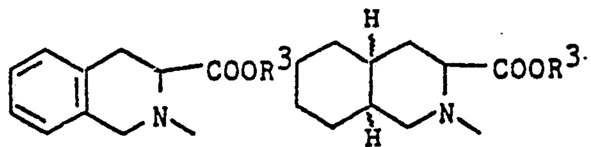
C D

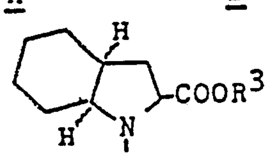
E

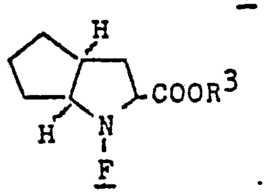
F

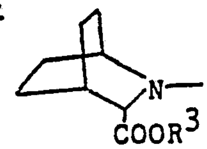
G

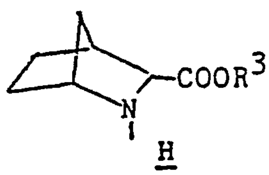
H

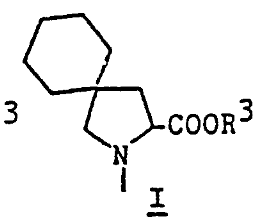
I

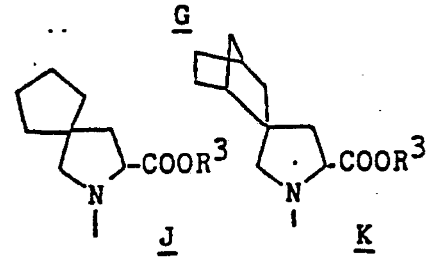
J K

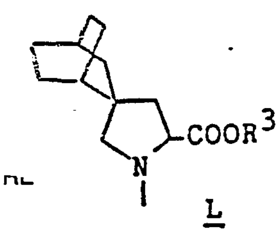
L

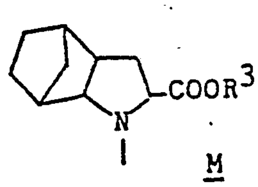
M

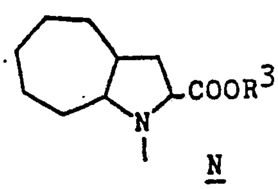
N

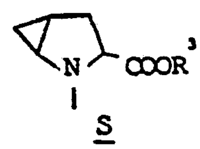
S

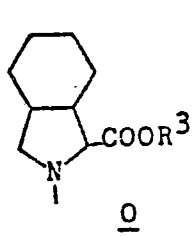
O

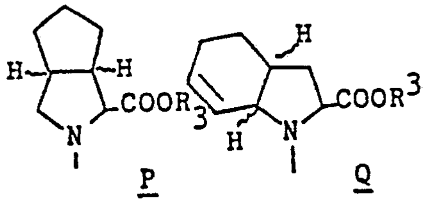
P Q

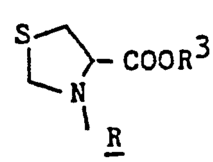
R

Zur Herstellung von Carbonsäureamid- und Peptidbindungen ist eine große Anzahl von Verfahren bekannt (siehe z. B. Houben-Weyl, Methoden der organischen Chemie, Bd. XV, Teil II, S. 1-364. Ferner Angew. Chemie 92, 129 (1980)). Alle diese Verfahren zielen mit unterschiedlichem Erfolg darauf, die für die Synthese von Peptiden notwendigen Kriterien der Racemisierungsfreiheit, der einfachen und schonenden Durchführbarkeit bei hohen Ausbeuten und mit leicht zugänglichen, möglichst ungefährlichen Ausgangsmaterialien sicherzustellen.

Die Ausbeuten bei den bisher bekannten Methoden zur Herstellung von Verbindungen der Formel I ausgehend von Verbindungen der Formeln II und IV (z. B. der HOBt-DCCl-Methode mit DMF oder Acetonitril als Lösungsmittel) liegen bei 50-75 %. Im Falle des DCCl bereitet die restlose Entfernung des entstehenden Dicyclohexylharnstoffs Schwierigkeiten, außerdem sind schwere Allergien gegen Carbodiimide bekannt. Andere Reagentien, z. B. andere Anhydride von Phosphorsäuren sind prinzipiell geeignet, das HOBt-Verfahren zu ersetzen, jedoch gilt es zu reaktive Reagentien zu vermeiden, um Nebenreaktionen (beispielsweise mit der ungeschützten sekundären Aminogruppe in der Verbindung der Formel II) zu verhindern.

Das vorliegende Verfahren stellt einen neuen Weg dar, die genannten Bedingungen für eine wirtschaftliche Synthese von Verbindungen der Formel I zu otpimieren. Mit Hilfe des erfindungsgemäßen Verfahrens lassen sich Verbindungen der Formel II mit solchen der Formel IV unter milden Bedingungen in guter Ausbeute zu Verbindungen der Formel I umsetzen. Es ist überraschend, daß es dabei nicht zu Nebenreaktionen an der ungeschützten sekundären Aminogruppe in den Verbindungen der Formel II bzw. dem Endprodukt kommt.

Die zum Schutz der funktionellen Gruppen eingeführten Reste werden anschließend in üblicher Weise abgespalten.

Bevorzugt sind solche Phosphinsäureanhydride der Formel III, in welcher $R^6$ bis $R^9$ für ($C_1$ und $C_{10}$)-Alkyl und/oder ($C_7$-$C_{10}$)-Aralkyl (z. B. Benzyl) stehen. Bevorzugt sind weiterhin Anhydride, bei denen beide P-Atome gleich substituiert sind.

Besonders geeignet im Sinne der Erfindung sind Anhydride der Formel III, in denen $R^6$ bis $R^9$ jeweils ein niedriges Alkyl ist, vorzugsweise ein solches mit 1 bis 4 C-Atomen.

Die erfindungsgemäß verwendeten Phosphinsäureanhydride sind farblose Flüssigkeiten. Sie sind bei Raumtemperatur stabil und lassen sich bei vermindertem Druck unzersetzt destillieren. In den meisten Lösungsmitteln, insbesondere in Lipidlösungsmitteln wie Chloroform oder Methylenchlorid, aber auch in polaren Lösungsmitteln wie DMF, DMA und Wasser sind sie löslich ($C_1$-$C_3$)-Alkylverbindungen. In Wasser findet langsame Zersetzung statt.

Als Anhydride der Dialkylphosphinsäuren seien beispielsweise genannt: Methylpropylphosphinsäureanhydrid, Methyl-butylphosphinsäureanhydrid, Diäthylphosphinsäureanhydrid, Di-n-propylphosphinsäureanhydrid, Di-n-butylphosphinsäureanhydrid, insbesondere Methylethylphosphinsäureanhydrid.

Die Herstellung der Dialkylphosphinsäureanhydride kann in an sich bekannter Weise erfolgen, z. B. durch Umsetzung der Dialkylphosphinsäurechloride mit Dialkylphosphinsäurealkylestern bei 150-160 °C (Houben-Weyl, Methoden der Organischen Chemie, G. Thieme Verl., Stuttgart 1963, Bd. XII, S. 266 ff). Besonders bevorzugt sind Verfahren, bei denen Dialkylphosphinsäure, deren Salze oder Ester mit Phosgen umgesetzt werden (DE-PS 2 129 583, DE-OS-2 225 545).

Während der Umsetzung werden gegebenenfalls in den Resten R bis $R^5$ vorhandene freie Amino-, Alkylamino, Hydroxy-, Carboxy-, Mercapto- und/oder Guanidinofunktionen in an sich bekannter Weise blockiert (vgl. z. B. Kontakte Merck 3/79, S. 14 ff. und 1/80, S. 23 ff.).

Besonders bevorzugt ist eine Ausführungsform, die dadurch gekennzeichnet ist, daß Verbindungen der Formel I hergestellt werden, in der

n = 1 oder 2 ist,

R ($C_1$ bis $C_6$)-Alkyl, ($C_2$ bis $C_6$)-Alkenyl, ($C_3$ bis $C_9$)-Cycloalkyl, Amino-($C_1$-$C_4$)-alkyl, ($C_2$-$C_5$)-Acylamino-($C_1$-$C_4$)-alkyl, ($C_7$-$C_{13}$)-Aroylamino-($C_1$-$C_4$)-alkyl, ($C_1$-$C_4$)-Alkoxycarbonylamino-($C_1$-$C_4$)-alkyl, ($C_6$ bis $C_{12}$)-Aryl-($C_1$-$C_4$)-alkoxycarbonylamino-($C_1$-$C_4$)-alkyl, ($C_6$ bis $C_{12}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkylamino und/oder Methylendioxy mono-, di- oder trisubstituiert sein kann, oder 3-Indolyl, insbesondere Methyl, Ethyl, Cyclohexyl, tert. Butoxycarbonylamino-($C_1$-$C_4$)-alkyl, Benzoyloxycarbonylamino-($C_1$-$C_4$)-alkyl oder Phenyl, das durch Phenyl, ($C_1$ bis $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)alkyl-amino, Nitro und/oder Methylendioxy mono- oder disubstituiert oder im Falle von Methoxy, trisubstituiert sein kann, bedeutet,

$R^1$ Wasserstoff oder ($C_1$ bis $C_6$)-Alkyl, das gegebenenfalls durch Amino, ($C_1$ bis $C_6$)-Acylamino oder Benzoylamino substituiert sein kann, ($C_2$ bis $C_6$)-Alkenyl, ($C_3$ bis $C_9$)-Cycloalkyl, ($C_5$ bis $C_9$)-Cycloalkenyl, ($C_3$ bis $C_7$)-Cycloalkyl-($C_1$ bis $C_4$)-alkyl, ($C_6$ bis $C_{12}$)-Aryl oder teilhydriertes Aryl, das jeweils durch ($C_1$ bis $C_4$)-Alkyl, ($C_1$ oder $C_2$)-Alkoxy oder Halogen substituiert sein kann, ($C_6$-$C_{12}$)-Aryl-($C_1$ bis $C_4$)-alkyl oder ($C_7$-$C_{13}$)-Aroyl-($C_1$-$C_2$)alkyl die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder 1 bis 4 Ringatome Stickstoffatome darstellen, oder eine Seitenkette einer natürlich vorkommenden, gegebenenfalls geschützten α-Aminosäure, insbesondere aber Wasserstoff, ($C_1$ bis $C_3$)-Alkyl, ($C_2$ oder $C_3$)-Alkenyl, die gegebenenfalls geschützte Seitenkette

von Lysin, Benzyl, 4-Methoxybenzyl, 4-Ethoxybenzyl, Phenethyl, 4-Amino-butyl oder Benzoylmethyl bedeutet,

$R^2$ und $R^3$ gleiche oder verschiedene Reste Wasserstoff, ($C_1$ bis $C_6$)-Alkyl, ($C_2$ bis $C_6$)-Alkenyl oder ($C_6$ bis $C_{12}$)-Aryl-($C_1$ bis $C_4$)-alkyl, insbesondere aber Wasserstoff, ($C_1$ bis $C_4$)-Alkyl oder Benzyl bedeuten und

$R^4$ und $R^5$ die oben angegebene Bedeutung haben, wobei andere funktionelle Gruppen während der Umsetzung, wie oben beschrieben, blockiert werden.

Insbesondere bevorzugt ist beispielsweise ein Verfahren, das zu Verbindungen der Formel I führt, in welcher

n = 2 ist, R = Phenyl, $R^1$ = Methyl,

$R^2$ und $R^3$ gleiche oder verschiedene ($C_1$ bis $C_6$)-Alkyl-Reste oder ($C_7$ bis $C_{10}$)-Aralkyl-Reste (wie Benzyl oder Nitrobenzyl) bedeuten und

$R^4$ und $R^5$ zusammen für ein Rest der Formel steht,

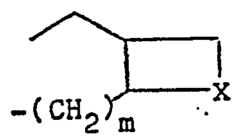

worin m = 0 oder 1 und X =, eine Bindung, $-CH_2-CH_2-$, $-CH_2-CH_2-CH_2-$ oder $-CH=CH-CH_2-$ bedeuten, wobei ein mit X gebildeter 6-Ring auch ein Benzolring sein kann.

Unter Aryl ist hier wie im folgenden vorzugsweise gegebenenfalls substituiertes Phenyl, Biphenylyl oder Naphthyl zu verstehen. Entsprechendes gilt für von Aryl abgeleitete Reste wie Aryloxy, Arylthio. Unter Aroyl wird insbesondere Benzoyl verstanden. Aliphatische Reste können geradkettig oder verzweigt sein.

Unter einem mono- bzw. bicyclischen Heterocyclen-Rest mit 5 bis 7 bzw. 8 bis 10 Ringatomen, wovon 1 bis 2 Ringatome Schwefel oder Sauerstoffatome und/oder wovon 1 bis 4 Ringatome Stickstoffatome darstellen, wird beispielsweise Thienyl, Benzo [b] thienyl, Furyl, Pyranyl, Benzofuryl, Pyrrolyl, Imidazolyl, Pyrazolyl, Pyridyl, Pyrimidinyl, Pyridazinyl, Indazolyl, Isoindolyl, Indolyl, Purinyl, Chinolizinyl, Isochinolinyl, Phthalazinyl, Naphthyridinyl, Chinoxalinyl, Chinazolyl, Cinnolinyl, Pteridinyl, Oxazolyl, Isoxazolyl, Thiazolyl oder Isothiazolyl verstanden. Diese Reste können auch teilweise oder vollständig hydriert sein.

Natürlich vorkommende α-Aminosäuren sind z. B. in Houben-Weyl, Methoden der Organischen Chemie, Bd. XV/1 und XV/2 beschrieben.

Falls $R^1$ für eine Seitenkette einer geschützten natürlich vorkommenden α-Aminosäure steht, wie z. B. geschütztes Ser, Thr, Asp, Asn, Glu, Gln, Arg, Lys, Hyl, Cys, Orn, Cit, Tyr, Trp, His oder Hyp, sind als Schutzgruppen die in der Peptidchemie üblichen Gruppen bevorzugt (vgl. Houben-Weyl, Bd. XV/1 und XV/2). Im Falle, daß $R^1$ die geschützte Lysin-Seitenkette bedeutet, werden die bekannten Amino-Schutzgruppen, insbesondere aber Z, Boc oder ($C_1$-$C_6$)-Alkanoyl bevorzugt. Als O-Schutzgruppen für Tyrosin kommen bevorzugt ($C_1$-$C_6$)-Alkyl, insbesondere Methyl oder Ethyl in Frage.

Nach dem erfindungsgemäßen Verfahren können, je nachdem, welche chiralen Ausgangsverbindungen verwendet wurden, Verbindungen der Formel I erhalten werden, in denen die Chiralitätszentren in der S- und/oder R-Konfiguration oder racemisch vorliegen.

Besonders vorteilhaft können die folgenden Verbindungen nach dem erfindungsgemäßen Verfahren erhalten werden.

N-(1-S-Carbethoxy-3-phenyl-propyl)-S-alanyl-S-prolinbenzylester
N-(1-R-Carbethoxy-3-phenyl-propyl)-S-alanyl-S-prolinbenzylester
N-(1-R,S-Carbethoxy-3-phenyl-propyl)-S-alanyl-S-prolinbenzylester
N-(1-S-Carbethoxy-3-cyclohexyl-propyl)-S-alanyl-S-prolinbenzylester
N-(1-R-Carbethoxy-3-cyclohexyl-propyl)-S-alanyl-S-prolinbenzylester
N-(1-R,S-Carbethoxy-3-cyclohexyl-propyl)-S-alanyl-S-prolinbenzylester
N-(1-S-Carbethoxy-3-phenyl-propyl)-$N_\varepsilon$-benzyloxycarbonyl-S-lysyl-S-prolin-tert. butylester
N-(1-S-Carbethoxy-3-phenyl-propyl)-S-tyrosyl-S-prolin benzylester
N-(1-S-Carbethoxy-3-phenyl-propyl)-O-methyl-S-tyrosyl-S-prolin-benzylester
N-(1-S-Carbethoxy-3-phenyl-propyl)-O-ethyl-S-tyrosyl-S-prolin-benzylester
N-(1-S-Carbethoxy-3-phenyl-propyl)-S-alanyl-S-pipecolsäure-benzylester
N-(1-S-Carbethoxy-3-cyclohexyl-propyl)-S-alanyl-S-pipecolsäurebenzylester
N-(1-S-Carbethoxy-3-phenyl-propyl)-S-alanyl-S-1,2,3,4-tetrahydroisochinolin-3-carbonsäure-tert. butylester
N-(1-S-Carbethoxy-3-cyclohexyl-propyl)-S-alanyl-S-1,2,3,4-tetrahydroisochinolin-3-carbonsäure-tert. butylester
N-(1-S-Carbethoxy-3-phenyl-propyl)-$N_\varepsilon$-benzylcarbonyl-S-lysyl-S-1,2,3,4-tetrahydroisochinolin-3-carbonsäure-tert. butylester
N-(1-S-Carbethoxy-3-phenyl-propyl)-O-ethyl-S-tyrosyl-S-1,2,3,4-tetrahydroisochinolin-3-carbonsäure-tert. butylester

N-(1-S-Carbethoxy-3-phenyl-propyl)-S-alanyl-3S-decahydroisochinolin-3-carbonsäure-benzylester

N-(1-S-Carbethoxy-3-phenyl-propyl)-S-alanyl-(2S,3aS,7aS)-octahydroindol-2-carbonsäure-benzylester

N-(1-S-Carbethoxy-3-cyclohexyl-propyl)-S-alanyl-(2S,3aS,7aS)-octahydroindol-2-carbonsäure-benzylester

N-(1-S-Carbethoxy-3-phenyl-propyl)-$N_\varepsilon$-benzyloxycarbonyl-S-lysyl-(2S,3aS,7aS)-octahydroindol-2-carbonsäure-tert. butylester

N-(1-S-Carbethoxy-3-cyclohexyl-propyl)-$N_\varepsilon$-benzyloxycarbonyl-S-lysyl-(2S,3aS,7aS)-octahydroindol-2-carbonsäure-tert. butylester

N-(1-S-Carbethoxy-3-phenyl-propyl)-O-methyl-S-tyrosyl-(2S,3aS,7aS)-octahydroindol-2-carbonsäure-benzylester

N-(1-S-Carbethoxy-3-phenyl-propyl)-O-ethyl-S-tyrosyl-(2S,3aS,7aS)-octahydroindol-2-carbonsäure-benzylester

N-(1-S-Carbethoxy-4,4-dimethylphenyl)-O-alanyl-(2S-3aS,7aS)-octahydroindol-2-carbonsäure-benzylester

N-[1-S-Carbethoxy-3-(4-fluorphenyl)-propyl]-S-alanyl-(2S,3aS,7aS)-octahydroindol-2-carbonsäure-benzylester

N-[1-S-Carbethoxy-3-(4-methoxyphenyl)-propyl]-S-alanyl-(2S,3aS,7aS)-octahydroindol-2-carbonsäure-benzylester

N-[1-S-Carbethoxy-3-(3,4-dimethoxyphenyl)-propyl]-S-alanyl-(2S,3aS,7aS)-octahydroindol-2-carbonsäure-benzylester

N-(1-S-Carbethoxy-3-cyclopentylpropyl)-S-alanyl-(2S,3aS,7aS)-octahydroindol-2-carbonsäure benzylester

N-(1-S-Carbethoxy-3-phenyl-propyl)-S-alanyl-(2S,3aR,7aS)-octahydroindol-2-carbonsäure-benzylester

N-(1-S-Carbethoxy-3-cyclohexyl-propyl)-S-alanyl-(2S-3aR-7aS)-octahydroindol-2-carbonsäure benzylester

N-(1-S-Carbethoxy-3-phenyl-propyl)-$N_\varepsilon$-benzyloxycarbonyl-S-lysyl(2S,3aR,7aS)-octahydroindol-2-carbonsäure tert. butylester

N-(1-S-Carbethoxy-3-cyclohexyl-propyl)-$N_\varepsilon$-benzyloxycarbonyl-S-lysyl-(2S,3aR,7aS)-octahydroindol-2-carbonsäure-benzylester

N-(1-S-Carbethoxy-3-phenyl-propyl)-O-ethyl-S-tyrosyl-(2S,3aR,7aS)-octahydroindol-2-carbonsäure-benzylester

N-(1-S-Carbethoxy-3-phenyl-propyl)-S-alanyl-(2S,3aR,7aR)-octahydroindol-2-carbonsäure-benzylester

N-(1-S-Carbethoxy-3-phenyl-propyl)-$N_\varepsilon$-benzyloxycarbonyl-S-lysyl-(2S-3aR,7aR)-octahydroindol-2-carbonsäure-tert. butylester

N-(1-S-Carbethoxy-3-cyclohexyl-propyl)-S-alanyl-(2S,3aR,7aR)-octahydroindol-2-carbonsäure-benzylester

N-(1-S-Carbethoxy-3-cyclohexyl-propyl)-O-ethyl-S-tyrosyl-(2S,3aR,7aR)-octahydroindol-2-carbonsäure-benzylester

N-(1-S-Carbethoxy-3-phenyl-propyl)-S-alanyl-(2S,3aS,7aR)-octahydroindol-2-carbonsäure-benzylester

N-(1-S-Carbethoxy-3-phenyl-propyl)-O-ethyl-S-tyrosyl-(2S,3aS,7aS)-octahydroindol-2-carbonsäure-benzylester

N-(1-S-Carbethoxy-4,4-dimethylphenyl)-O-alanyl-(2S,3aS,7aS)-octahydroindol-2-carbonsäure-benzylester

N-[1-S-Carbethoxy-3-(4-fluorphenyl)-propyl]-S-alanyl-(2S,3aS,7aS)-octahydroindol-2-carbonsäure-benzylester

N-[1-S-Carbethoxy-3-(4-methoxyphenyl)-propyl]-S-alanyl-(2S,3aS,7aS)-octahydroindol-2-carbonsäure-benzylester

N-[1-S-Carbethoxy-3-(3,4-dimethoxyphenyl)-propyl]-S-alanyl-(2S-3aS-7aS)-octahydroindol-2-carbonsäure-benzylester

N-(1-S-Carbethoxy-3-cyclopentylpropyl)-S-alanyl-(2S-3aS-7aS)-octahydroindol-2-carbonsäure benzylester

N-(1-S-Carbethoxy-3-phenyl-propyl)-$N_\varepsilon$-benzyloxycarbonyl-S-lysyl-(2S,3aR,6aS)-octahydrocyclopenta[b]pyrrol-2-carbonsäure-tert. butylester

N-(1-S-Carbethoxy-3-cylohexylpropyl)-$N_\varepsilon$-benzyloxycarbonyl-S-lysyl-(2S,3aR,6aS)-octahydrocylopenta[b]pyrrol-2-carbonsäure-tert. butylester

N-(1-S-Carbethoxy-3-phenyl-propyl)-O-ethyl-S-tyrosyl-(2S-3aR,6aS)-octahydrocylopenta[b]pyrrol-2-carbonsäure-benzylester

N-(1-S-Carbethoxy-3-cyclopentyl-propyl)-S-alanyl-2-(2S,3aR,6aS)-octahydrocyclopenta[b]pyrrol-2-carbonsäure-benzylester

N-(1-S-Carbethoxy-3-phenyl-propyl)-S-alanyl-S-2-azabicylo[2.2.2]-octan-3-carbonsäure-benzylester

N-(1-S-Carbethoxy-3-phenyl-propyl)-O-ethyl-S-tyrosyl-S-2-azabicyclo[2.2.2]-octan-3-carbonsäure-benzylester

N-(1-S-Carbethoxy-3-cyclohexyl-propyl)-S-alanyl-S-2-azabicyclo[2.2.2]-octan-3-carbonsäure-benzylester

N-(1-S-Carbethoxy-3-cyclohexyl-propyl)-$N_ε$-benzyloxycarbonyl-S-2-azabicylo[2.2.2]-octan-3-carbonsäure-tert. butylester

N-(1-S-Carbethoxy-3-phenyl-propyl)-$N_ε$-benzyloxycarbonyl-S-lysyl-S-2-azabicyclo[2.2.2]-octan-3-carbonsäure-tert. butylester

N-(1-S-Carbethoxy-3-cyclopentylpropyl)-S-alanyl-S-2-azabicyclo[2.2.2]-octan-3-carbonsäure-benzylester

N-(1-S-Carbethoxy-3-phenyl-propyl)-S-alanyl-3S-exo-2-azabicyclo[2.2.1]-heptan-3-carbonsäure-benzylester

N-(1-S-Carbethoxy-3-cyclohexylpropyl)-S-alanyl-3S-exo-2-azabicyclo[2.2.1]-heptan-3-carbonsäure-benzylester

N-(1-S-Carbethoxy-3-phenyl-propyl)-$N_ε$-benzyloxycarbonyl-S-lysyl-3S-exo-2-azabicyclo[2.2.1]-heptan-3-carbonsäure-tert. butylester

N-(1-S-Carbethoxy-3-phenyl-propyl)-S-alanyl-3S-endo-2-azabicyclo[2.2.1]-heptan-3-carbonsäure-benzylester

N-(1-S-Carbethoxy-3-cyclohexylpropyl)-S-alanyl-3S-endo-2-azabicyclo[2.2.1]-heptan-3-carbonsäure-benzylester

N-(1-S-Carbethoxy-3-phenyl-propyl)-$N_ε$-benzyloxycarbonyl-S-lysyl-3S-endo-2-azabicyclo[2.2.1]-heptan-3-carbonsäure tert. butylester

N-(1-S-Carbethoxy-3-phenyl-propyl)-O-ethyl-S-tyrosyl-3S-endo-2-azabicyclo[2.2.1]-heptan-3-carbonsäure-benzylester

N-(1-S-Carbethoxy-3-phenyl-propyl)-S-alanyl-2-azaspiro[4,5]decan-3-S-carbonsäure-benzylester

N-(1-S-Carbethoxy-3-phenyl-propyl)-O-ethyl-S-tyrosyl-2-azaspiro[4,5]decan-3-S-carbonsäure-benzylester

N-(1-S-Carbethoxy-3-phenyl-propyl)-$N_ε$-benzyloxycarbonyl-S-lysyl-2-azaspiro[4,5]decan-3-S-carbonsäure-tert. butylester

N-(1-S-Carbethoxy-3-cyclohexylpropyl)-S-alanyl-2-azaspiro[4,5]decan-3-S-carbonsäure-benzylester

N-(1-S-Carbethoxy-3-cylohexylpropyl)-$N_ε$-benzyloxycarbonyl-S-lysyl-2-azaspiro[4,5]decan-3-S-carbonsäure-tert. butylester

N-(1-S-Carbethoxy-3-phenyl-propyl)-S-alanyl-2-azaspiro[4,4]nonan-3-S-carbonsäure-benzylester

N-(1-S-Carbethoxy-3-phenyl-propyl)-O-ethyl-S-tyrosyl-2-azaspiro[4,4]nonan-3-S-carbonsäure benzylester

N-(1-S-Carbethoxy-3-phenyl-propyl)-$N_ε$-tert. butoxycarbonyl-S-lysyl-2-azaspiro[4,4]nonan-3-S-carbonsäure-benzylester

N-(1-S-Carbethoxy-3-cyclohexyl-propyl)-S-alanyl-2-azaspiro[4,4]nonan-3-S-carbonsäure-benzylester

N-(1-S-Carbethoxy-3-cyclopentyl-propyl)-S-alanyl-2-azaspiro[4,4]nonan-3-S-carbonsäure-benzylester

N-(1-S-Carbethoxy-3-cyclopentyl-propyl)-$N_ε$-tert. butoxycarbonyl-S-lysyl-2-azaspiro[4,4]nonan-3-S-carbonsäure-benzylester

N-(1-S-Carbethoxy-3-phenyl-propyl)-S-alanyl-spiro [bicyclo[2.2.1]heptan 2,3′-pyrrolidin]-5′-S-carbonsäure-benzylester

N-(1-S-Carbethoxy-3-phenyl-propyl)-O-ethyl-S-tyrosyl-spiro [bicyclo[2.2.1]heptan-2,3′-pyrrolidin]-5′-S-carbonsäure-benzylester

N-(1-S-Carbethoxy-3-phenyl-propyl)-$N_ε$-benzyloxycarbonyl-S-lysyl-spiro [bicyclo[2.2.1]heptan-2,3′-pyrrolidin]-5′-S-carbonsäure-tert. butylester

N-(1-S-Carbethoxy-3-cyclohexyl-propyl)-S-alanyl-spiro [bicyclo[2.2.1]heptan-2,3′-pyrrolidin]-5′-S-carbonsäure-benzylester

N-(1-S-Carbethoxy-3-cyclohexyl-propyl)-$N_ε$-tert. butoxycarbonyl-S-lysyl-spiro [bicyclo[2.2.1]heptan 2,3′-pyrrolidin]-5′-S-carbonsäure-benzylester

N-(1-S-Carbethoxy-3-phenyl-propyl)-S-alanyl-spiro [bicyclo[2.2.2]octan-2,3′-pyrrolidin]-5′-S-carbonsäure-benzylester

N-(1-S-Carbethoxy-3-phenyl-propyl)-O-ethyl-S-tyrosyl-spiro [bicyclo[2.2.2]octan-2,3′-pyrrolidin]-5′-S-carbonsäure-benzylester

N-(1-S-Carbethoxy-3-phenyl-propyl)-$N_ε$-tert. butoxycarbonyl-S-lysyl spiro [bicyclo[2.2.2]octan-2,3′-pyrrolidin]-5′-S-carbonsäure-benzylester

N-(1-S-Carbethoxy-3-cyclohexyl-propyl)-S-alanyl-spiro [bicyclo[2.2.2]octan-2,3′-pyrrolidin]-5′-S-carbonsäure-benzylester

N-(1-S-Carbethoxy-3-phenyl-propyl)-S-alanyl-azatricyclo [4,3,0,$1^{6,9}$]decan-3-S-carbonsäure-benzylester

N-(1-S-Carbethoxy-3-phenyl-propyl)-O-ethyl-S-tyrosyl-2-azatricyclo [4,3,0,$1^{6,9}$]decan-3-S-carbonsäure-benzylester

N-(1-S-Carbethoxy-3-phenyl-propyl)-$N_ε$-benzyloxycarbonyl-S-lysyl-2-azatricyclo [4,3,0,$1^{6,9}$]decan-3-S-carbonsäure-tert. butylester

N-(1-S-Carbethoxy-3-cyclohexyl-propyl)-S-alanyl-2-azatricyclo-[4,3,0,1$^{6,9}$]decan 3-S-carbonsäure-benzylester

N-(1-S-Carbethoxy-3-phenyl-propyl)-$N_\varepsilon$-benzyloxycarbonyl-S-lysyl-2-azatricyclo [4,3,0,1$^{6,9}$]decan-3-S-carbonsäure tert. butylester

N-(1-S-Carbethoxy-3-phenyl-propyl)-S-alanyl-decahydrocyclohepta[b]pyrrol-2-S-carbonsäure-benzylester

N-(1-S-Carbethoxy-3-phenyl-propyl)-O-ethyl-S-tyrosyldecahydrocyclohepta[b]pyrrol-2-S-carbonsäure-benzylester

N-(1-S-Carbethoxy-3-phenyl-propyl)-$N_\varepsilon$-benzyloxycarbonyl-S-lysyl-decahydrocyclohepta[b]pyrrol-2-S-carbonsäure-tert. butylester

N-(1-S-Carbethoxy-3-cyclohexyl-propyl)-S-alanyl-decahydrocyclohepta[b]pyrrol-2-S-carbonsäure-benzylester

N-(1-S-Carbethoxy-3-cyclohexyl-propyl)-$N_\varepsilon$-tert. butoxycarbonyl-S-lysyl-decahydrocyclohepta[b]pyrrol-2-S-carbonsäure-benzylester

N-(1-S-Carbethoxy-3-phenyl-propyl)-S-alanyl-trans-octahydroisoindol-1-S-carbonsäure-benzylester

N-(1-S-Carbethoxy-3-phenyl-propyl)-S-alanyl-cis-octahydroisoindol-1-S-carbonsäure-benzylester

N-(1-S-Carbethoxy-3-cyclohexyl-propyl)-S-alanyl-trans-octahydroisoindol-1-S-carbonsäure-benzylester

N-(1-S-Carbethoxy-3-cyclohexyl-propyl)-S-alanyl-cis-octahydroisoindol-1-S-carbonsäure-benzylester

N-(1-S-Carbethoxy-3-phenyl-propyl)-S-alanyl-cis-octahydrocyclopenta[c]pyrrol-1-S-carbonsäure-benzylester

N-(1-S-Carbethoxy-3-cyclohexyl-propyl)-S-alanyl-cis-octahydrocylopenta[c]pyrrol-1-S-carbonsäure-benzylester

N-(1-S-Carbethoxy-3-cyclohexyl-propyl)-$N_\varepsilon$-tert. butoxycarbonyl-S-lysyl-cis-octahydrocyclopenta[c]pyrrol-1-S-carbonsäure-benzylester

N-(1-S-Carbethoxy-3-phenyl-propyl)-S-alanyl-2,3,3a,4,5,7a-hexahydroindol-cis-endo-2-S-carbonsäure-tert. butylester

N-(1-S-Carbethoxy-3-cyclohexyl-propyl)-O-ethyl-S-tyrosyl-2,3,3a,4,5,7a-hexahydroindol-2-S-carbonsäure-tert. butylester

N-(1-S-Carbethoxy-3-cyclohexyl-propyl)-S-alanyl-2,3,3a,4,5,7a-hexahydroindol-2-S-carbonsäure-tert. butylester

N-(1-S-Carbethoxy-3-phenyl-propyl)-S-alanyl-thiazolidin-5-S-carbonsäure-tert. butylester

N-(1-S-Carbethoxy-3-cyclohexyl-propyl)-S-alanyl-thiazolidin-5-S-carbonsäure-tert. butylester

N-(1-S-Carbethoxy-3-phenyl-propyl)-$N_\varepsilon$-benzyloxycarbonyl-lysyl-thiazolidin-5-S-carbonsäure-tert. butylester

N-(1-S-Carbethoxy-3-cyclohexyl-propyl)-$N_\varepsilon$-benzyloxycarbonyl-S-2-azabicyclo[3.1.0]-hexan-3-carbonsäure-tert. butylester

N-(1-S-Carbethoxy-3-phenyl-propyl)-$N_\varepsilon$-benzyloxycarbonyl-S-lysyl-S-2-azabicyclo[3.1.0]-hexan-3-carbonsäure-tert. butylester

N-(1-S-Carbethoxy-3-cyclopentylpropyl)-S-alanyl-S-2-azabicyclo[3.1.0]-hexan-3-carbonsäure-benzylester

Die erfindungsgemäße Umsetzung wird vorzugsweise in neutralem oder schwach alkalischen Medium durchgeführt. Am einfachsten ist es, den pH-Wert des Medium durch Zugabe von aliphatischen oder cycloaliphatischen tertiären Basen wie N-Methylmorpholin, N-Ethylmorpholin oder Trialkylaminen mit bis zu 6 C-Atomen pro Alkylrest einzustellen. Beim bevorzugten Arbeiten in zweiphasigwäßrigen Systemen können anstelle der organischen Base auch als Puffersysteme wirkende alkalische Salze z. B. solche der Kohlensäure oder der Phosphorsäure verwendet werden.

Als Lösungsmittel können alle in der Peptidsynthese üblichen inerten Lösungsmittel, z. B. Methylenchlorid, Essigester Chloroform, Dimethylformamid, Dimethylacetamid, Dioxan oder Tetrahydrofuran Verwendung finden.

Die Synthese läßt sich auch in gemischtwäßrigen Lösungsmittelsystem durchführen. Hierunter werden Gemische aus Wasser und einem mit Wasser mischbaren organischen Lösungsmittel wie Dioxan/Wasser, Tetrahydrofuran/Wasser oder Dimethylformamid/Wasser verstanden. Überraschenderweise sind zweiphasige Systeme, wie $CH_2Cl_2$/Wasser, Essigester/Wasser oder 3-Methyltetrahydropyran/Wasser mit $Na_2CO_3$ oder $K_2CO_3$ als Base besonders geeignet (gutes Rühren erforderlich).

Die Reaktion läuft in der Regel bei Raumtemperatur hinreichend schnell ab. Leichtes Erwärmen schadet nicht. Höhere Temperaturen, etwa oberhalb 50 °C, sind wegen der Racemisierungsgefahr nicht zu empfehlen, und unnötig.

Die erfindungsgemäßen Phosphinsäureanhydride werden vorzugsweise im Überschuß (etwa 1,2-2,5 Mol Phosphinsäureanhydrid/Mol zu knüpfender Bindung) eingesetzt. Sie können als solche unverdünnt oder in dem organischen Lösungsmittel gelöst dem Reaktionsgemisch zugetropft werden.

Ethylmethylphosphinsäureanhydrid eignet sich im besonderem Maße, da es sowohl in Wasser als auch in organischen Lösungsmittel löslich ist und die mit ihr aus Carbonsäuren gebildeten gemischten Anhydride bei Raumtemperatur die gewünschte mittlere Aktivität aufweisen. Diese Verbindung kann

besonders vorteilhaft im « Eintopfverfahren » eingesetzt werden, da keinerlei Reaktion des Anhydrids mit Aminogruppen beobachtet wurde. Das in situ in Gegenwart von Basen aus II gebildete gemischte Anhydrid VI reagiert ausschließlich und in fast quantitativer Ausbeute mit den Aminosäureestern der Formel IV.

Die mittlere Aktivität des gemischten Anhydrids wirkt sich bei technischen Ansätzen weiterhin dadurch vorteilhaft aus, daß nur eine geringe positive Wärmetönung vorhanden ist und Kühlprobleme nicht auftreten.

Eine besonders vorteilhafte Verfahrensvariante ergibt sich daraus, daß die Reaktion zweiphasig in Wasser une einem mit Wasser nicht oder nur begrenzt mischbaren Lösungsmittel wie z. B. $CH_2Cl_2$, AcOEt durchgeführt werden kann, in dem das Reaktionsprodukt (I) löslich ist, sich Ausgangsstoffe sowie weitere zugesetzte Reagentien (Salze von Org. tert. Basen, Soda, phosphinsaure Salze u. ä.) jedoch nur schlecht lösen.

Die Reaktion verläuft racemisierungsfrei ($<$ 2 %) und wird durch einen Wassergehalt von Lösungsmitteln und Reagentien nicht gestört, ja sie kann sogar in Wasser oder 2-phasigen Systemen unter Verwendung organischer (NEM, $Et_3N$ u. a.) oder bevorzugt anorganischen Basen (z. B. Soda) besonders vorteilhaft durchgeführt werden.

Bei Verwendung eines organischen oder gemischt-organischen Mediums kann die org. Phase nach der Abreaktion durch Ausschütteln mit wäßr. $KHSO_4/K_2SO_4$-Lösung (pH 2) und anschließend mit Soda-Bikarbonatlösung von Ausgangsstoffen sowie Verunreinigungen weitgehend befreit werden. Die entstehenden Produkte hinterbleiben als Öle nach Einengen der org. Phase und werden beispielsweise durch Hydrierung (z. B. im Falle $R^3$ = Benzyl, Nitrobenzyl) oder Säurebehandlung (z. B. wenn $R^3$ = $Bu^t$) in biolog. aktive Substanzen überführt.

Die Verbindungen der Formel I sind Hemmstoffe des Angiotensin Converting Enzymes (ACE) beziehungsweise Zwischenprodukte bei der Herstellung von solchen Hemmstoffen und können zur Bekämpfung des Bluthochdrucks verschiedener Genese eingesetzt werden. Solche Verbindungen sind beispielsweise bekannt aus US-PS 4 344 949, US-PS 4 374 847, US-PS 4 350 704, EP-A 50 800, EP-A 31 741, EP-A 51 020, EP-A 49 658, EP-A 49 605, EP-A 29 488, EP-A 46 953 und EP-A 52 870. Sie sind weiterhin Gegenstand der deutschen Patentanmeldungen P 32 26 768.1, P 31 51 690.4, P 32 10 496.0, P 32 11 397.8, P 32 11 676.4, P 32 27 055.0, P 32 42 151.6, P 32 46 503.3 und P 32 46 757.5.

Die nachfolgenden Beispiele sollen das erfindungsgemäße Verfahren erläutern, ohne die Erfindung auf die hier stellvertretend genannten Substanzen zu beschränken.

Beispiel 1

Homogenes Verfahren

85 g S,S,S-Azabicyclo-[3.3.0]octan-3-carbonsäurebenzylesterhydrochlorid und 88 g (S,N(1-Carboethoxy-3-phenyl-propyl)-alanin werden in 500 ml Methylenchlorid aufgeschlämmt. Man setzt in 1 Stunde unter Rühren 120 ml Ethylmethylphosphinsäureanhydrid (in Form einer 50 %igen Lösung in Methylenchlorid) und 200 ml Triethylamin zu. Nach 2 Stunden wird mit Hilfe der Dünnschichtchromatographie (Kieselgel-System : $CHCl_3$/MeOH/AcOH 50 + 10 + 3) der Umsatz kontrolliert. Ist der Aminosäurebenzylester noch nachweisbar werden weitere 20 ml Anhydrid und 50 ml $Et_3N$ zugefügt. Man beläßt einige Stunden bei Raumtemperatur (gegebenenfalls über Nacht), engt im Vakuum ein, verdünnt den Reaktionsansatz mit Methylenchlorid (500 ml) auf ca. 1 l Gesamtvolumen und extrahiert 1 × mit 500 ml Kaliumhydrogensulfat/Kaliumsulfat-Lösung (50 g $KHSO_4$ in 1 l Wasser), und 2 × mit je 500 ml einer wäßrigen 5 % $NaHCO_3$-Lösung. Die organische Phase wird über etwas festem Natriumsulfat getrocknet und über eine 2-3 cm dicke Schicht aus Kieselgel filtriert. Man wäscht mit 250 ml Methylenchlorid oder Essigester nach und engt das fast farblose Eluat ein. Ausbeute : 85-95 % d. Th. Zur Überprüfung von Ausbeute und Reinheit des Produktes kann nach beschriebenem Verfahren katalytisch entbenzyliert werden (Methanol/Pd/C). Das Reaktionsprodukt-((S)-N(1-Carboethoxy-3-phenyl-propyl)-alanyl-2-azabicyclo[3.3.0]octan-3-carbonsäure) kristallisiert aus Äther in 80-90 %iger Ausbeute (bezogen auf (S)-Azabicyclo[3.3.0]octan-3-carbonsäurebenzylester-hydrochlorid).

Schmp. : 109 °C ; $[\alpha]^{24}_D$ = + 15,8° (C = 1, Methanol).

Beispiel 2

Zweiphasiges Verfahren

28,2 g (S,S,S) Azabicylo[3.3.0]octan-3-carbonsäure-benzylester·HCl und 29,5 g (S)-N-(Carboethoxy-3-phenyl-1-propyl)-alanin werden in einer Mischung aus 150 ml Wasser und 100 ml Methylenchlorid aufgeschlämmt. Man fügt langsam unter kräftigem Rühren simultan eine Lösung von 70 g Kaliumcarbonat (oder eine äquivalente Menge Soda) in 150 ml Wasser und ca. 150 ml einer 25 %igen Lösung von Methylethylphosphinsäureanhydrid in Methylenchlorid bzw. eine entsprechende Menge MEPA in anderer Konzentration hinzu. Der pH-Wert soll zwischen 9 und 10 liegen ; die Zugabe ist in ca. 2 Stunden abgeschlossen. Die Vollständigkeit der Reaktion wird dünnschichtchromatographisch nach weiteren 2

Stunden durch Abwesenheit von Aminosäurebenzylester in der organischen Phase überprüft. Man verdünnt mit weiteren 250 ml Methylenchlorid und trennt die wäßrige Phase ab.

Die organische Phase wird 1 × mit 100 ml einer wäßrigen Lösung von 50 g $KHSO_4$ und 100 g$K_2SO_4$/Liter und anschließend 1 × mit 250 ml einer wäßrigen 5 %igen $NaHCO_3/Na_2CO_3$ Lösung extrahiert. Man trocknet die org. Phase über 10 g festem Natriumsulfat und engt im Vakuum ein. Das hinterbliebene farblose Öl kann wie im Beispiel 1 angegeben weiterverarbeitet werden. Ausbeute an kristallinem Endprodukt : 80-90 %. Das auf diesem Wege erhaltene Endprodukt stimmt in seinen physikalischen Daten mit dem im Beispiel 1 erhaltenen Produkte überein und zeichnet sich durch besondere Reinheit aus. (DC : Kieselgel-Fertigplatten [MERCK AG] $SiO_2$-60 ; System $CHCl_3$/MeOH/HOAc 50 : 10 : 5 (V : V)).

Die Basenzugabe kann auch mittels eines Autotitrators bei einem konstanten pH-Wert erfolgen.

Beispiel 3

Zweiphasiges Verfahren

29,0 [S,S,S]-Octahydroindol-2-carbonsäurebenzylester·HCl und 29,5 g (S)-N-3-phenyl-1-Carbethoxy-propyl-alanin werden in einer Mischung aus 200 ml Wasser und 100 ml Methylenchlorid aufgeschlämmt. Man fügt langsam unter kräftigem Rühren simultan eine Lösung von 70 g Kaliumcarbonat (oder eine äquivalente Menge Soda) in 150 ml Wasser und 150 ml einer 25 %igen Lösung von Methylethylphosphinsäureanhydrid in Methylenchlorid hinzu. Der pH-Wert soll zwischen 9 und 10 liegen ; Bei Verwendung von 3-Methyltetrahydropyran als org. Lösungsmittel kann bei pH 7-9 gearbeitet werden. Die Zugabe ist in ca. 2 Stunden abgeschlossen. Die Vollständigkeit der Reaktion wird dünnschichtchromatographisch nach weiteren 2 Stunden durch Abwesenheit von Aminosäurebenzylester in der organischen Phase überprüft. Man verdünnt mit weiteren 250 ml Methylenchlorid und trennt die wäßrige Phase ab.

Die organische Phase wird 1 × mit 100 ml einer wäßrigen Lösung von 50 g $KHSO_4$ und 100 g $K_2SO_4$/Liter und anschließend 1 × mit 250 ml einer wäßrigen 5 %igen $NaHCO_3/Na_2CO_3$ Lösung extrahiert. Man trocknet die org. Phase über 10 g festem Natriumsulfat und engt im Vakuum ein. Das hinterbliebene farblose Öl kann analog zu Beispiel 1 weiterverarbeitet werden.

Man überführt das so erhaltene Produkt mit HCl in das kristalline [S,S,S,S,S]-N-[(1-Carbethoxy-3-phenyl-propyl)-alanyl]-octahydroindol-2-carbonsäure-hydrochlorid, dessen physikalische Daten mit den aus der Literatur bekannten übereinstimmen.

## Patentansprüche

1. Verfahren zur Herstellung von Verbindungen der Formel I

$$R^3OOC-\underset{R^4}{\overset{*}{C}H}-\underset{R^5}{N}-\underset{O}{\overset{}{\underset{\|}{C}}}-\underset{R^1}{\overset{*}{C}H}-NH-\underset{COOR^2}{\overset{*}{C}H}-(CH_2)_n-R \qquad (I)$$

in welcher

n = 1 oder 2 ist,

R Wasserstoff, Alkyl mit 1-8 C-Atomen, Alkenyl mit 2-6 C-Atomen, Cycloalkyl mit 3-9 C-Atomen, Aryl mit 6-12 C-Atomen, das durch ($C_1$-$C_4$)-Alkyl, ($C_1$-$C_3$)-Alkoxy, Hydroxy, Halogen, Nitro, Amino, Aminomethyl, ($C_1$-$C_4$)-Alkylamino, Di-($C_1$-$C_4$)-alkylamino, ($C_1$-$C_4$)-Acylamino, vorzugsweise ($C_1$-$C_4$)-Alkanoylamino, Methylendioxy, Carboxy, Cyano und/oder Sulfamoyl mono-, di- oder trisubstituiert sein kann, Alkoxy mit 1-4 C-Atomen, Aryloxy mit 6-12 C-Atomen, das wie oben bei Aryl beschrieben substituiert sein kann, mono- bzw. bicyclisches Heteroaryloxy mit 5-7 bzw. 8-10 Ringatomen, wovon 1 bis 2 Ringatome Schwefel- oder Sauerstoffatome und/oder 1 bis 4 Ringatome Stickstoff darstellen, das wie oben bei Aryl beschrieben substituiert sein kann, Amino-($C_1$-$C_4$)-alkyl, ($C_1$-$C_4$)-Alkanoylamino-($C_1$-$C_4$)-alkyl, ($C_7$-$C_{13}$)-Aroylamino-($C_1$-$C_4$)-alkyl, ($C_1$-$C_4$)-Alkoxy-carbonylamino-($C_1$-$C_4$)-alkyl, ($C_6$-$C_{12}$)-Aryl-($C_1$-$C_4$)-alkoxycarbonylamino-($C_1$-$C_4$)-alkyl, ($C_6$-$C_{12}$)-Aryl-($C_1$-$C_4$)-alkylamino-($C_1$-$C_4$)-alkyl, ($C_1$-$C_4$)-Alkylamino-($C_1$-$C_4$)-alkyl, Di-($C_1$-$C_4$)-alkylamino-($C_1$-$C_4$)-alkyl, Guanidino-($C_1$-$C_4$)-alkyl, Imidazolyl, Indolyl, ($C_1$-$C_4$)-Alkylthio, ($C_1$-$C_4$)-Alkylthio-($C_1$-$C_4$)-alkyl, ($C_6$-$C_{12}$)-Arylthio-($C_1$-$C_4$)-alkyl, das im Arylteil wie oben bei Aryl beschrieben, substituiert sein kann, ($C_6$-$C_{12}$)-Aryl-($C_1$-$C_4$)-alkylthio, das im Arylteil wie oben bei Aryl beschrieben substituiert sein kann, Carboxy-($C_1$-$C_4$)-alkyl, Carboxy, Carbamoyl, Carbamoyl-($C_1$-$C_4$)-alkyl, ($C_1$-$C_4$)-Alkoxy-carbonyl-($C_1$-$C_4$)-alkyl, ($C_6$-$C_{12}$)-Aryloxy-($C_1$-$C_4$)-alkyl, das im Arylteil wie oben bei Aryl beschrieben substituiert sein kann oder ($C_6$-$C_{12}$)-Aryl-($C_1$-$C_4$)-alkoxy, das im Arylteil wie oben bei Aryl beschrieben substituiert sein kann,

$R^1$ Wasserstoff, Alkyl mit 1-6 C-Atomen, Alkenyl mit 2-6 C-Atomen, Alkinyl mit 2-6 C-Atomen, Cycloalkyl mit 3-9 C-Atomen, Cycloalkenyl mit 5-9 C-Atomen, ($C_3$-$C_9$)-Cycloalkyl-($C_1$-$C_4$)-alkyl, ($C_5$-$C_9$)-

Cycloalkenyl($C_1$-$C_4$)-alkyl, gegebenenfalls teilhydriertes Aryl mit 6-12 C-Atomen, das wie oben bei R beschrieben substituiert sein kann, ($C_6$-$C_{12}$)-Aryl-($C_1$-$C_4$)-alkyl oder ($C_7$-$C_{13}$)-Aroyl-($C_1$ oder $C_2$)-alkyl die beide wie das vorstehende Aryl substituiert sein können, mono- bzw. bicyclisches, gegebenenfalls teilhydriertes Heteroaryl mit 5-7 bzw. 8-10 Ringatomen, wovon 1 bis 2 Ringatome Schwefel- oder Sauerstoffatome und/oder 1 bis 4 Ringatome Stickstoffatome darstellen, das wie das vorstehende Aryl substituiert sein kann oder die gegebenenfalls geschützte Seitenkette einer natürlich vorkommenden α-Aminosäure $R^1$—$CH(NH_2)$—COOH bedeuten,

$R^2$ und $R^3$ gleich oder verschieden sind und Wasserstoff, Alkyl mit 1-6 C-Atomen, Alkenyl mit 2-6 C-Atomen, Di-($C_1$-$C_4$)-alkylamino-($C_1$-$C_4$)-alkyl, ($C_1$-$C_5$)-Alkanoyloxy-($C_1$-$C_4$)-alkyl, ($C_1$-$C_6$)-Alkoxy-carbonyloxy-($C_1$-$C_4$)-alkyl, ($C_7$-$C_{13}$)-Aroyloxy-($C_1$-$C_4$)-alkyl, ($C_6$-$C_{12}$)-Aryloxycarbonyloxy-($C_1$-$C_4$)-alkyl, Aryl mit 6-12 C-Atomen, ($C_6$-$C_{12}$)-Aryl-($C_1$-$C_4$)-alkyl, ($C_3$-$C_9$)-Cycloalkyl oder ($C_3$-$C_9$)-Cycloalkyl-($C_1$-$C_4$)-alkyl bedeuten und

$R^4$ und $R^5$ zusammen mit den sie tragenden Atomen ein heterocyclisches mono-, bi- oder tricyclisches Ringsystem mit 3 bis 15 C-Atomen bilden, dadurch gekennzeichnet, daß man Verbindungen der Formel II

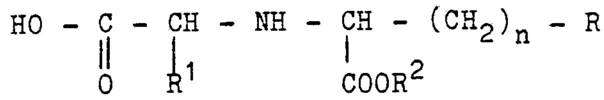

(II)

in welcher n, R, $R^1$ und $R^2$ die oben definierten Bedeutungen haben, in Gegenwart von Phosphinsäureanhydriden der Formel III

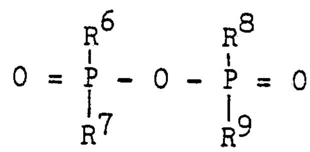

(III)

in welcher $R^6$, $R^7$, $R^8$ und $R^9$ gleich oder verschieden sind und für Alkyl und/oder Aralkyl stehen, mit Verbindungen der Formel IV

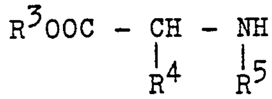

(IV)

in welcher $R^3$, $R^4$ und $R^5$ die oben definierten Bedeutungen haben, umsetzt, gegebenenfalls zum Schutz anderer funktioneller Gruppen eingeführte Reste abspaltet und gegebenenfalls freie Carboxygruppen in an sich bekannter Weise verestert.

2. Verfahren gemäß Anspruch 1, dadurch gekennzeichnet, daß Verbindungen der Formel I hergestellt werden, worin $R^4$ und $R^5$ zusammen mit den sie tragenden Atomen für ein System aus der Reihe Pyrrolidin, Piperidin, Tetrahydroisochinolin, Decahydroisochinolin, Octahydroindol, Octahydrocyclopenta [b] pyrrol, 2-Aza-bicyclo [2.2.2] octan, 2-Azabicyclo [2.2.1] heptan, 2-Azaspiro [4.5] decan, 2-Azaspiro [4.4] nonan, Spiro [(bicyclo [2.2.1] heptan)-2,3-pyrrolidin], Spiro [(bicyclo [2.2.2] octan)-2,3-pyrrolidin], 2-Azatricyclo [$4.3.0.1^{6,9}$] decan, Decahydrocyclohepta [b] pyrrol, Octahydroisoindol, Octahydrocyclopenta [c]-pyrrol, 2,3,3a,4,5,7a-Hexahydroindol, Tetrahydrothiazol und 2-Azabicyclo [3.1.0] hexan stehen.

3. Verfahren gemäß Anspruch 1 oder 2, dadurch gekennzeichnet, daß während der Umsetzung gegebenenfalls in den Resten R bis $R^5$ vorhandene freie Amino-, Alkylamino-, Hydroxy-, Carboxy-, Mercapto- und/oder Guanidinofunktionen in an sich bekannter Weise blockiert werden.

4. Verfahren gemäß einem der Ansprüche 1 bis 3, dadurch gekennzeichnet, daß in Gegenwart von Ethylmethylphosphinsäureanhydrid gearbeitet wird.

5. Verfahren gemäß einem der Ansprüche 1 bis 4, dadurch gekennzeichnet, daß die Umsetzung in einem inerten organischen Lösungsmittel in Gegenwart eines tert. organischen Amines durchgeführt wird.

6. Verfahren gemäß einem der Ansprüche 1 bis 4, dadurch gekennzeichnet, daß die Umsetzung mit Ethylmethylphosphinsäureanhydrid zweiphasig unter Verwendung einer wäßrigen anorganischen Base (bei pH 7-10) und einem mit Wasser nicht oder nur begrenzt mischbaren organischen Lösungsmittel wie z. B. Methylenchlorid, Essigester oder 3-Methyltetrahydropyran durchgeführt wird.

7. Verfahren gemäß einem der Ansprüche 1 bis 6, dadurch gekennzeichnet, daß [S,S,S,S,S]-N-[(1-Carbethoxy-3-phenyl-propyl)-alanyl]-octahydroindol-2-carbonsäurebenzylester hergestellt wird.

8. Verfahren gemäß einem der Ansprüche 1 bis 6, dadurch gekennzeichnet, daß N-[(1(S)-Carbethoxy-3-phenyl-propyl)-(S)-alanyl]-3aR, 7aS-octahydroindol-2-(S)-carbonsäurebenzylester hergestellt wird.

9. Verfahren gemäß einem der Ansprüche 1 bis 6, dadurch gekennzeichnet, daß [S,S,S,S,S]-N-[(1-Carbethoxy-3-phenyl-propyl)-alanyl]-decahydroisochinolin-3-carbonsäurebenzylester hergestellt wird.

10. Verfahren gemäß einem der Ansprüche 1 bis 6, dadurch gekennzeichnet, daß [S,S,S]-N-[(1-Carbethoxy-3-phenyl-propyl)-alanyl]-tetrahydroisochinolin-3-carbonsäurebenzylester hergestellt wird.

11. Verfahren gemäß einem der Ansprüche 7 bis 10, dadurch gekennzeichnet, daß anstelle der Benzylester die entsprechenden tert. Butylester hergestellt werden.

12. Verfahren gemäß einem der Ansprüche 1 bis 4, dadurch gekennzeichnet, daß die Umsetzung zweiphasig unter Verwendung einer wäßrigen anorganischen Base und eines mit Wasser nicht oder nur begrenzt mischbaren organischen Lösungsmittels durchgeführt wird, wobei die Basenzugabe mit Hilfe eines pH-gesteuerten Autotitrators erfolgt.

13. Verfahren gemäß einem der Ansprüche 1 bis 6, dadurch gekennzeichnet, daß (S)-N-(1-Carbethoxy-3-phenylpropyl)-alanyl-[S,S,S]-2-aza-bicyclo [3.3.0] octan-3-carbonsäure-benzylester hergestellt wird.

## Claims

1. A process for the preparation of compounds of the formula I

$$R^3OOC-\overset{*}{\underset{R^4}{CH}}-\underset{R^5}{N}-\underset{O}{\overset{\|}{C}}-\overset{*}{\underset{R^1}{CH}}-NH-\overset{*}{\underset{COOR^2}{CH}}-(CH_2)_n-R \qquad (I)$$

in which n is 1 or 2, R denotes hydrogen, alkyl having 1-8 carbon atoms, alkenyl having 2-6 carbon atoms, cycloalkyl having 3-9 carbon atoms, aryl which has 6-12 carbon atoms and can be mono-, di- or trisubstituted by $(C_1-C_4)$-alkyl, $(C_1-C_3)$-alkoxy, hydroxyl, halogen, nitro, amino, aminomethyl, $(C_1-C_4)$-alkylamino, di-$(C_1-C_4)$-alkylamino, $(C_1-C_4)$-acylamino, preferably $(C_1-C_4)$-alkanoylamino, methylenedioxy, carboxyl, cyano and/or sulfamoyl, alkoxy having 1-4 carbon atoms, aryloxy which has 6-12 carbon atoms and can be substituted as described above for aryl, mono- or bicyclic heteroaryloxy which has 5-7 or 8-10 ring atoms respectively, 1 to 2 of the ring atoms representing sulfur or oxygen atoms and/or 1 to 4 ring atoms representing nitrogen, and which can be substituted as described above for aryl, amino-$(C_1-C_4)$-alkyl, $(C_1-C_4)$-alkanoyl-amino-$(C_1-C_4)$-alkyl, $(C_7-C_{13})$-aroylamino-$(C_1-C_4)$-alkyl, $(C_1-C_4)$-alkoxy-carbonylamino-$(C_1-C_4)$-alkyl, $(C_6-C_{12})$-aryl-$(C_1-C_4)$-alkoxycarbonylamino-$(C_1-C_4)$-alkyl, $(C_6-C_{12})$-aryl-$(C_1-C_4)$-alkylamino-$(C_1-C_4)$-alkyl, $(C_1-C_4)$-alkylamino-$(C_1-C_4)$-alkyl, di-$(C_1-C_4)$-alkylamino-$(C_1-C_4)$-alkyl, guanidino-$(C_1-C_4)$-alkyl, imidazolyl, indolyl, $(C_1-C_4)$-alkylthio, $(C_1-C_4)$-alkylthio-$(C_1-C_4)$-alkyl, $(C_6-C_{12})$-arylthio-$(C_1-C_4)$-alkyl which can be substituted in the aryl moiety as described above for aryl, $(C_6-C_{12})$-aryl-$(C_1-C_4)$-alkylthio which can be substituted in the aryl moiety as described above for aryl, carboxyl-$(C_1-C_4)$-alkyl, carboxyl, carbamoyl, carbamoyl-$(C_1-C_4)$-alkyl, $(C_1-C_4)$-alkoxy-carbonyl-$(C_1-C_4)$-alkyl, $(C_6-C_{12})$-aryloxy-$(C_1-C_4)$-alkyl which can be substituted in the aryl moiety as described above for aryl, or $(C_6-C_{12})$-aryl-$(C_1-C_4)$-alkoxy which can be substituted in the aryl moiety as described above for aryl, $R^1$ denotes hydrogen, alkyl having 1-6 carbon atoms, alkenyl having 2-6 carbon atoms, alkynyl having 2-6 carbon atoms, cycloalkyl having 3-9 carbon atoms, cycloalkenyl having 5-9 carbon atoms, $(C_3-C_9)$-cycloalkyl-$(C_1-C_4)$-alkyl, $(C_5-C_9)$-cycloalkenyl$(C_1-C_4)$-alkyl, optionally partially hydrogenated aryl which has 6-12 carbon atoms and can be substituted as described above for R, $(C_6-C_{12})$-aryl-$(C_1-C_4)$-alkyl or $(C_7-C_{13})$-aroyl-($C_1$ or $C_2$)-alkyl, both of which can be substituted as the preceding aryl, mono- or bicyclic, optionally partially hydrogenated heteroaryl which has 5-7 or 8-10 ring atoms respectively, 1 to 2 of these ring atoms representing sulfur or oxygen atoms and/or 1 to 4 ring atoms representing nitrogen atoms, which can be substituted as the preceding aryl, or, the protected side chain of a naturally occurring α-amino acid $R^1$—$CH(NH_2)$—COOH, $R^2$ and $R^3$ are identical or different and denote hydrogen, alkyl having 1-6 carbon atoms, alkenyl having 2-6 carbon atoms, di-$(C_1-C_4)$-alkylamino-$(C_1-C_4)$-alkyl, $(C_1-C_5)$-alkanoyloxy-$(C_1-C_4)$-alkyl, $(C_1-C_6)$-alkoxy-carbonyloxy-$(C_1-C_4)$-alkyl, $(C_7-C_{13})$-aroyloxy-$(C_1-C_4)$-alkyl, $(C_6-C_{12})$-aryloxycarbonyloxy-$(C_1-C_4)$-alkyl, aryl having 6-12 carbon atoms, $(C_6-C_{12})$-aryl-$(C_1-C_4)$-alkyl, $(C_3-C_9)$-cycloalkyl or $(C_3-C_9)$-cycloalkyl-$(C_1-C_4)$-alkyl, and $R^4$ and $R^5$, together with the atoms carrying them, form a heterocyclic mono-, bi- or tricyclic ring system having 3 to 15 carbon atoms, which comprises compounds of the formula II

$$HO-\underset{O}{\overset{\|}{C}}-\underset{R^1}{CH}-NH-\underset{COOR^2}{CH}-(CH_2)_n-R \qquad (II)$$

in which n, R, $R^1$ and $R^2$ have the meanings defined above, being reacted in the presence of a phosphinic anhydride of the formula III

$$\begin{array}{ccccccccc} & & R^6 & & & & R^8 & & \\ & & | & & & & | & & \\ O & = & P & - & O & - & P & = & O \\ & & | & & & & | & & \\ & & R^7 & & & & R^9 & & \end{array} \qquad \text{(III)}$$

in which $R^6$, $R^7$, $R^8$ and $R^9$ are identical or different and represent alkyl and/or aralkyl, with compounds of the formula IV

$$\begin{array}{ccccc} R^3OOC & - & CH & - & NH \\ & & | & & | \\ & & R^4 & & R^5 \end{array} \qquad \text{(IV)}$$

in which $R^3$, $R^4$ and $R^5$ have the meanings defined above, where appropriate radicals which have been introduced to protect other functional groups being eliminated and, where appropriate, free carboxyl groups being esterified in a manner known per se.

2. The process as claimed in claim 1, wherein compounds of the formula I in which $R^4$ and $R^5$, together with the atoms carrying them, represent a system from the series comprising pyrrolidine, piperidine, tetrahydroisoquinoline, decahydroisoquinoline, octahydroindole, octahydrocyclopenta [b] pyrrole, 2-aza-bicyclo [2.2.2] octane, 2-azabicyclo [2.2.1] heptane, 2-azaspiro [4.5] decane, 2-azasprio [4.4] nonane, spiro [(bicyclo [2.2.1] heptane)-2,3-pyrrolidine], spiro [(bicyclo [2.2.2] octane)-2,3-pyrrolidine], 2-azatricyclo [4.3.0.$1^{6,9}$] decane, decahydrocyclohepta [b]-pyrrole, octahydroisoindole, octahydrocyclopenta [c]-pyrrole, 2,3,3a,4,5,7a-hexahydroindole, tetrahydrothiazole and 2-azabicyclo [3.1.0] hexane are prepared.

3. The process as claimed in claim 1 or 2, wherein during the reaction the free amino, alkylamino, hydroxyl, carboxyl, mercapto and/or guanidino groups present where appropriate in the radicals R to $R^5$ are protected in a manner known per se.

4. The process as claimed in any of claims 1 to 3, which is carried out in the presence of ethylmethylphosphinic anhydride.

5. The process as claimed in any of claims 1 to 4, wherein the reaction is carried out in an inert organic solvent in the presence of a tert. organic amine.

6. The process as claimed in any of claims 1 to 4, wherein the reaction with ethylmethylphosphinic anhydride is carried out in two phases using an aqueous inorganic base (at pH 7-10) and an organic solvent which is immiscible, or miscible to only a limited extent, with water, such as, for example, methylene chloride, ethyl acetate or 3-methyltetrahydropyrane.

7. The process as claimed in any of claims 1 to 6, wherein benzyl [S,S,S,S,S]-N-[(1-carboethoxy-3-phenylpropyl)alanyl] octahydroindole-2-carboxylate is prepared.

8. The process as claimed in any of claims 1 to 6, wherein benzyl N-[(1(S)-carboethoxy-3-phenylpropyl)-(S)-alanyl]-3aR,7aS-octahydroindole-2-(S)-carboxylate is prepared.

9. The process as claimed in any of claims 1 to 6, wherein benzyl [S,S,S,S,S]-N-[(1-carboethoxy-3-phenylpropyl)alanyl] decahydroisoquinoline-3-carboxylate is prepared.

10. The process as claimed in any of claims 1 to 6, wherein benzyl [S,S,S]-N-[(1-carboethoxy-3-phenylpropyl)-alanyl] tetrahydroisoquinoline-3-carboxylate is prepared.

11. The process as claimed in any of claims 7 to 10, wherein the tert.-butyl ester is prepared in place of the corresponding benzyl ester.

12. The process as claimed in any of claims 1 to 4, wherein the reaction is carried out in two phases using an aqueous inorganic base and an organic solvent which is immiscible, or miscible to only a limited extent, with water, the addition of base being carried out using a pH-controlled autotitrator.

13. The process as claimed in any of claims 1 to 6, wherein benzyl (S)-N-(1-carbethoxy-3-phenylpropyl)alanyl-[S,S,S]-2-azabicyclo [3.3.0] octane-3-carboxylate is prepared.

**Revendications**

1. Procédé pour la préparation de composés de formule I

$$\begin{array}{ccccccccccccccc} & & * & & & & & & * & & & & * & & \\ R^3OOC & - & CH & - & N & - & C & - & CH & - & NH & - & CH & - & (CH_2)_n - R \\ & & | & & | & & \| & & | & & & & | & & \\ & & R^4 & & R^5 & & O & & R^1 & & & & COOR^2 & & \end{array} \qquad \text{(I)}$$

dans laquelle

n est égal à 1 ou 2,

R représente l'hydrogène, un groupe alcoyle ayant de 1 à 8 atomes de carbone, un groupe alcényle ayant de 2 à 6 atomes de carbone, un groupe cycloalcoyle ayant de 3 à 9 atomes de carbone, un groupe aryle ayant de 6 à 12 atomes de carbone, qui peut être mono-, di- ou tri-substitué par un groupe alcoyle en $C_1$-$C_4$, alcoxy en $C_1$-$C_4$, hydroxy, un halogène, un groupe nitro, amino, aminométhyle, alcoyl($C_1$-$C_4$)-amino, di-alcoyl($C_1$-$C_4$)-amino, acyl($C_1$-$C_4$)-amino, de préférence alcanoyl($C_1$-$C_4$)-amino, méthylène dioxy, carboxy, cyano et/ou sulfamoyle, un groupe alcoxy ayant de 1 à 4 atomes de carbone, un groupe aryloxy ayant de 6 à 12 atomes de carbone, qui peut être substitué tel que décrit pour le groupe aryle, un groupe hétéroaryloxy mono- ou bicyclique ayant de 5 à 7 ou de 8 à 10 atomes dans le noyau, dont de 1 à 2 atomes du noyau représentent des atomes de soufre ou d'oxygène et/ou de 1 à 4 atomes du noyau représentent des atomes d'azote, qui peut être substitué tel que décrit pour le groupe aryle, un groupe amino-alcoyle en $C_1$-$C_4$, un groupe alcanoyl($C_1$-$C_4$)-amino-alcoyle($C_1$-$C_4$), un groupe aroyl($C_7$-$C_{13}$)-amino-alcoyle($C_1$-$C_4$), un groupe alcoxy($C_1$-$C_4$)-carbonylamino-alcoyle($C_1$-$C_4$), un groupe aryl($C_6$-$C_{12}$)-alcoxy($C_1$-$C_4$)-carbonylamino-alcoyle($C_1$-$C_4$), un groupe aryl($C_6$-$C_{12}$)-alcoyl($C_1$-$C_4$)-amino-alcoyle($C_1$-$C_4$), un groupe alcoyl($C_1$-$C_4$)-amino-alcoyle($C_1$-$C_4$), un groupe di-alcoyl($C_1$-$C_4$)-amino-alcoyle($C_1$-$C_4$), un groupe guanidino-alcoyle($C_1$-$C_4$), un groupe imidazolyle, indolyle, un groupe alcoyl($C_1$-$C_4$)-thio, un groupe alcoyl($C_1$-$C_4$)-thio-alcoyle($C_1$-$C_4$), un groupe aryl($C_6$-$C_{12}$)-thio-alcoyle($C_1$-$C_4$), qui peut être substitué dans la partie aryle, tel que décrit ci-dessus pour le groupe aryle, un groupe aryl($C_6$-$C_{12}$)-alcoyl($C_1$-$C_4$)-thio, qui peut être substitué dans la partie aryle, tel que décrit ci-dessus pour le groupe aryle, un groupe carboxy-alcoyle($C_1$-$C_4$), un groupe carboxy, carbamoyle, un groupe carbamoyl-alcoyle($C_1$-$C_4$), un groupe alcoxy($C_1$-$C_4$)-carbonyl-alcoyle($C_1$-$C_4$), un groupe aryloxy($C_6$-$C_{12}$)-alcoyle($C_1$-$C_4$), qui peut être substitué dans la partie aryle, tel que décrit ci-dessus pour le groupe aryle, un groupe aryl($C_6$-$C_{12}$)-alcoxy($C_1$-$C_4$), qui peut être substitué dans la partie aryle, tel que décrit pour le groupe aryle.

$R^1$ représente l'hydrogène, un groupe alcoyle ayant de 1 à 6 atomes de carbone, un groupe alcényle ayant de 2 à 6 atomes de carbone, un groupe alcynyle ayant de 2 à 6 atomes de carbone, un groupe cycloalcoyle ayant de 3 à 9 atomes de carbone, un groupe cycloalcényle ayant de 5 à 9 atomes de carbone, un groupe cycloalcoyl($C_3$-$C_9$)-alcoyle($C_1$-$C_4$), un groupe cycloalcényl($C_5$-$C_9$)-alcoyle($C_1$-$C_4$), un groupe aryle éventuellement partiellement hydrogéné, ayant de 6 à 12 atomes de carbone, qui peut être substitué tel que décrit ci-dessus pour R, un groupe aryl($C_6$-$C_{12}$)-alcoyle($C_1$-$C_4$) ou aroyl, ($C_7$-$C_{13}$)-alcoyle($C_1$ ou $C_2$), qui peuvent être tous les deux substitués comme le groupe aryle précédent, un groupe hétéroaryle mono- ou bicyclique, éventuellement partiellement hydrogéné, ayant de 5 à 7 ou 8 à 10 atomes dans le noyau, dont de 1 à 2 atomes du noyau représentent des atomes de soufre ou d'oxygène et/ou de 1 à 4 atomes du noyau représentent des atomes d'azote, qui peut être substitué comme le groupe aryle précédent, ou la chaîne latérale, éventuellement protégée, d'un acide α-aminé d'origine naturelle, $R^1$—$CH(NH_2)$—COOH,

$R^2$ et $R^3$ sont identiques ou différents et représentent l'hydrogène, un groupe alcoyle ayant de 1 à 6 atomes de carbone, un groupe alcényle ayant de 2 à 6 atomes de carbone, un groupe di-alcoyl($C_1$-$C_4$)-amino-alcoyle($C_1$-$C_4$), un groupe alcanoyloxy($C_1$-$C_5$)-alcoyle($C_1$-$C_4$), un groupe alcoxy($C_1$-$C_6$)-carbonyloxy-alcoyle($C_1$-$C_4$), un groupe aroyloxy($C_7$-$C_{13}$)-alcoyle($C_1$-$C_4$), un groupe aryloxy($C_6$-$C_{12}$)-carbonyloxy-alcoyle($C_1$-$C_4$), un groupe aryle ayant de 6 à 12 atomes de carbone, un groupe aryl($C_6$-$C_{12}$)-alcoyle($C_1$-$C_4$), un groupe cycloalcoyle en $C_3$-$C_9$ ou un groupe cycloalcoyl($C_3$-$C_9$)-alcoyle($C_1$-$C_4$), et

$R^4$ et $R^5$ forment ensemble avec les atomes les portant un système hétérocyclique mono-, bi- ou tricyclique ayant de 3 à 15 atomes de carbone, lequel procédé est caractérisé en ce qu'on fait réagir des composés de formule II

$$\begin{array}{ccccccccccccc} HO & - & \underset{\underset{O}{\|}}{C} & - & \underset{\underset{R^1}{|}}{CH} & - & NH & - & \underset{\underset{COOR^2}{|}}{CH} & - & (CH_2)_n & - & R \end{array} \qquad (II)$$

dans laquelle n, R, $R^1$ et $R^2$ ont les significations définies ci-dessus, en présence d'anhydrides d'acides phosphiniques de formule III

$$O = \overset{\overset{R^6}{|}}{\underset{\underset{R^7}{|}}{P}} - O - \overset{\overset{R^8}{|}}{\underset{\underset{R^9}{|}}{P}} = O \qquad (III)$$

dans laquelle $R^6$, $R^7$, $R^8$ et $R^9$ sont identiques ou différents et représentent un groupe alcoyle ou aralcoyle, avec des composés de formule IV

$$R^3OOC - \underset{R^4}{\underset{|}{CH}} - \underset{R^5}{\underset{|}{NH}} \qquad (IV)$$

dans laquelle $R^3$, $R^4$ et $R^5$ ont les significations définies ci-dessus, on élimine éventuellement les groupes introduits pour la protection d'autres fonctions et on estérifie éventuellement les groupes carboxy libres d'une manière connue en soi.

2. Procédé selon la revendication 1, caractérisé en ce qu'on prépare des composés de formule I, dans lesquels $R^4$ et $R^5$ forment ensemble avec les atomes les portant, un système éventuellement substitué de la série des : pyrrolidine, pipéridine, tétrahydroisoquinoléine, décahydroisoquinoléine, octahydroindole, octahydrocyclopenta [b]-pyrrole, 2-aza-bicyclo [2.2.2] octane, 2-azabicyclo [2.2.1] heptane, 2-azaspiro [4.5] décane, 2-azaspiro [4.4] nonane, spiro [(bicyclo [2.2.1] heptane)-2,3-pyrrolidine], spiro [(bicyclo [2.2.2] octane)-2,3-pyrrolidine], 2-azatricyclo [4,3,0,$1^{6,9}$] décane, décahydrocycloheptane-[b] pyrrole, octahydroisoindole, octahydrocyclopenta [c]-pyrrole, 2,3,3a,4,5,7a-hexahydroindole, tétrahydrothiazole et 2-azabicyclo [3.1.0] hexane.

3. Procédé selon la revendication 1 ou la revendication 2, caractérisé en ce que pendant la réaction on bloque les fonctions amino, alcoylamino, hydroxy ; carboxy, mercapto et/ou guanidino, libres, éventuellement présentes dans les radicaux R à $R^5$, d'une manière connue en soi.

4. Procédé selon l'une quelconque des revendications 1 à 3, caractérisé en ce qu'on travaille en présence d'anhydride de l'acide éthylméthylphosphinique.

5. Procédé selon l'une quelconque des revendications 1 à 4, caractérisé en ce que la réaction est conduite dans un solvant organique inerte, en présence d'une amine organique tertiaire.

6. Procédé selon l'une quelconque des revendications 1 à 4, caractérisé en ce que la réaction est effectuée avec de l'anhydride de l'acide éthylméthylphosphinique, en deux phases, en utilisant une base inorganique aqueuse (pH de 7 à 10) et un solvant organique non miscible ou seulement peu miscible avec l'eau, tel que par exemple le chlorure de méthylène, l'acétate d'éthyle ou le 3-méthyltétrahydropyrane.

7. Procédé selon l'une quelconque des revendications 1 à 6, caractérisé en ce qu'on prépare l'ester benzylique de l'acide [S,S,S,S,S]-N-[(1-carbéthoxy-3-phényl-propyl)-alanyl]-octahydroindole-2-carboxylique.

8. Procédé selon l'une quelconque des revendications 1 à 6, caractérisé en ce qu'on prépare l'ester benzylique de l'acide N-[(1(S)-carbéthoxy-3-phényl-propyl)-(S)-alanyl]-3aR, 7aS-octahydroindole-2-(S)-carboxylique.

9. Procédé selon l'une quelconque des revendications 1 à 6, caractérisé en ce qu'on prépare l'ester benzylique de l'acide [S,S,S,S,S]-N-[(1-carbéthoxy-3-phényl-propyl)-alanyl]-décahydroisoquinoléine-3-carboxylique.

10. Procédé selon l'une quelconque des revendications 1 à 6, caractérisé en ce qu'on prépare l'ester benzylique de l'acide [S,S,S]-N-[(1-carbéthoxy-3-phényl-propyl)-alanyl]-tétrahydroisoquinoléine-3-carboxylique.

11. Procédé selon l'une quelconque des revendications 7 à 10, caractérisé en ce qu'au lieu de l'ester benzylique, on prépare l'ester tert-butylique correspondant.

12. Procédé selon l'une quelconque des revendications 1 à 4, caractérisé en ce que la réaction a lieu en deux phases, en utilisant une base inorganique aqueuse et un solvant organique non miscible ou seulement peu miscible avec l'eau, l'addition de base étant effectuée à l'aide d'un auto-titreur commandé par le pH.

13. Procédé selon l'une quelconque des revendications 1 à 6, caractérisé en ce que l'on prépare l'ester benzylique de l'acide (S)-N-(1-carbethoxy-3-phényl-propyl)-alanyl-[S,S,S]-2-aza-bicyclo [3.3.0] octane-3-carboxylique.